# EUROPEAN PATENT APPLICATION

(11) **EP 3 626 153 A1**
(43) Date of publication of application: **25.03.2020**
(21) Application number: 19198756.9
(22) Date of filing: 20.09.2019
(51) Int. Cl.: A61B 1/00, A61B 1/05, A61B 5/00, A61B 5/01, A61B 1/12

(54) **SURGICAL IMAGING SYSTEM AND METHODS OF USE THEREOF**

(30) Priority: 21.09.2018 US 201862734272 P; 06.08.2019 US 201916532830
(71) Applicant: Covidien LP, Mansfield, MA 02048 (US)
(72) Inventor: VARADHAN, Sridharan, 500019 Hyderabad (IN); POOLA, Ananth, 500044 Hyderabad (IN); VADALI, K V S Manoj Kumar, 502032 Hyderabad (IN)
(74) Representative: Maschio, Antonio

(57) **Abstract**

A surgical imaging system includes an endoscope, a processor, and a display. The endoscope includes an elongated shaft having a distal end portion, an imager attached to the distal end portion and configured to capture an image of tissue, an infrared transmitter attached to the distal end portion of the shaft for transmitting infrared light to tissue, and an infrared receiver attached to the distal end portion of the shaft for receiving infrared light reflected by the tissue. The processor is in communication with the infrared transmitter and the infrared receiver and is configured to identify generate a digital image of vasculature in the tissue based on data received from the infrared receiver. The display is configured to display the image of the tissue captured by the imager with the digital image of the vasculature overlaying the image of the tissue.

## Description

### CROSS-REFERENCE TO RELATED APPLICATION

This application claims the benefit of and priority to U.S. Provisional Patent Application No. 62/734,272 filed September 21, 2018, the entire disclosure of which is incorporated by reference herein.

### BACKGROUND

### 1. Technical Field

The present disclosure relates to minimally-invasive surgery. More particularly, the present disclosure relates to methods and apparatus for imaging vasculature during minimally-invasive surgical procedures.

### 2. Background of Related Art

Surgical techniques and instruments have been developed that allow the surgeon to perform an increasing range of surgical procedures with minimal incisions into the skin and body tissue of the patient. Minimally-invasive surgery has become widely accepted in many medical specialties, often replacing traditional open surgery. Unlike open surgery, which requires a long incision, minimally-invasive procedures, such as endoscopy or laparoscopy, are performed through one or more short incisions, with much less trauma to the body.

In laparoscopic and endoscopic surgical procedures, a small "keyhole" incision or puncture is made in a patient's body, e.g., in the abdomen, to provide an entry point for a surgical access device which is inserted into the incision and facilitates the insertion of specialized instruments used in performing surgical procedures within an internal surgical site. The number of incisions may depend on the type of surgery. It is not uncommon for some abdominal operations, e.g., gallbladder surgery, to be performed through a single incision. In most patients, the minimally-invasive approach leads to decreased postoperative pain, shorter hospital stay, faster recovery, decreased incidence of wound-related and pulmonary complications, cost savings by reducing post-operative care, and, in some cases, a better overall outcome.

In minimally-invasive surgery, the surgeon does not have direct visualization of the surgical field, and thus minimally-invasive techniques require specialized skills compared to the corresponding open surgical techniques. Although minimally-invasive techniques vary widely, surgeons generally rely on a lighted camera at the tip of an endoscope to view the surgical site, with a monitor displaying a magnified version of the site for the surgeon to use as a reference during the surgical procedure. The surgeon then performs the surgery while visualizing the procedure on the monitor. During surgical procedures that require the identification and dissection of vasculature, it is often difficult for the surgeon to visualize the vasculature even with the assistance of the lighted camera. Accordingly, a need exists for an improved means of visualizing vasculature during minimally-invasive surgery.

### SUMMARY

In one aspect of the present disclosure, a surgical imaging system is provided and includes an endoscope, a processor, and a display. The endoscope includes an elongated shaft having a distal end portion, an imager attached to the distal end portion and is configured to capture an image of tissue, an infrared transmitter attached to the distal end portion of the shaft for transmitting infrared light to tissue, and an infrared receiver attached to the distal end portion of the shaft for receiving infrared light reflected by the tissue. The processor is in communication with the infrared transmitter and the infrared receiver and is configured to identify vasculature in the tissue and generate a digital image of the vasculature based on data received from the infrared receiver. The display is configured to display the image of the tissue captured by the imager and the digital image of the vasculature generated by the processor. The digital image of the vasculature overlays the image of the tissue.

In aspects, the image of the tissue captured by the imager may include an image of the vasculature. The display may be configured to superimpose the digital image of the vasculature generated by the processor on the image of the vasculature captured by the imager.

In some aspects, the image of the vasculature may be displayed in a color different than an actual color of the vasculature.

In other aspects, the color of the digital image of the vasculature may change based on a temperature of the vasculature.

In another aspect of the present disclosure, a method of imaging vasculature during a surgical procedure is provided and includes positioning a distal end portion of an endoscope adjacent tissue in a surgical site, capturing an image of the tissue with an imager attached to the distal end portion of the endoscope, transmitting infrared light to the tissue from an infrared transmitter attached to the distal end portion of the shaft, receiving the infrared light that is reflected by the tissue into an infrared receiver that is attached to the distal end portion of the shaft, generating a digital image of vasculature in the tissue using data from the infrared receiver, displaying the image of the tissue captured by the imager on a display, and displaying the digital image of the vasculature on the display over the image of the tissue.

In aspects, the method may further include identifying a location of the vasculature with a processor. The processor may generate the digital image of the vasculature using the data from the infrared receiver.

In some aspects, the image of the tissue captured by the imager may include an image of the vasculature. The display may be configured to superimpose the digital image of the vasculature generated on the image of the vasculature captured by the imager.

In further aspects, the method may further include displaying the digital image of the vasculature on the display in a color different than an actual color of the vasculature.

In other aspects, the color of the digital image of the vasculature may change based on a temperature of the vasculature.

Further details and aspects of exemplary embodiments of the present disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Embodiments of the present disclosure are described herein with reference to the accompanying drawings, wherein:
FIG. 1 is a schematic diagram of a surgical imaging system including an endoscope, a processor, and a display; a port assembly coupled to the endoscope; and a surgical instrument in accordance with an embodiment of the present disclosure;
FIG. 2 is a bottom view of the endoscope of the surgical imaging system of FIG. 1 illustrating an imager and infrared sensors;
FIG. 3A is a front view of a display of the surgical imaging system of FIG. 1 illustrating an actual image of tissue and vasculature at a surgical site; and
FIG. 3B is a front view of the display of FIG. 1 illustrating the actual image of the tissue and the vasculature with a digital image of the vasculature superimposed thereon.

### DETAILED DESCRIPTION

Embodiments of the presently disclosed surgical imaging system and methods of use thereof are described in detail with reference to the drawings, in which like reference numerals designate identical or corresponding elements in each of the several views. As used herein the term "distal" refers to that portion of the surgical imaging system or component thereof, that is closer to a patient, while the term "proximal" refers to that portion of the surgical imaging system or component thereof, that is farther from the patient.

As will be described in detail below, provided is a surgical imaging system including an endoscope having a camera for capturing images of tissue in a surgical site and infrared light transmitters and sensors for detecting and imaging vasculature disposed underneath the surface of the tissue. The endoscope is in communication with or includes a processor that generates a digital image of the vasculature (e.g., veins) using the data gathered by the infrared sensors. The processor is in communication with a display configured to display an actual image of the tissue and vasculature captured by the camera and superimposes the digital image of the vasculature over the actual image of the vasculature.

The present disclosure utilizes near-infrared (NIR) light to image or visualize to various depths, such as several cm, within tissue. Veins contain de-oxygenated hemoglobin, which has a near infrared absorption peak at around 760 nm and a lesser, more broad absorption plateau over the range of 800 to 950 nm. There is a window of wavelengths in the near infrared region between 650 and 900 nm in which photons are able to penetrate tissue far enough to illuminate deeper structures beyond depths of 1 cm. The surgical imaging system of the present disclosure takes advantage of this phenomenon by using near-infrared wavelengths of approximately 880 to 890 nm for imaging subcutaneous veins in tissue.

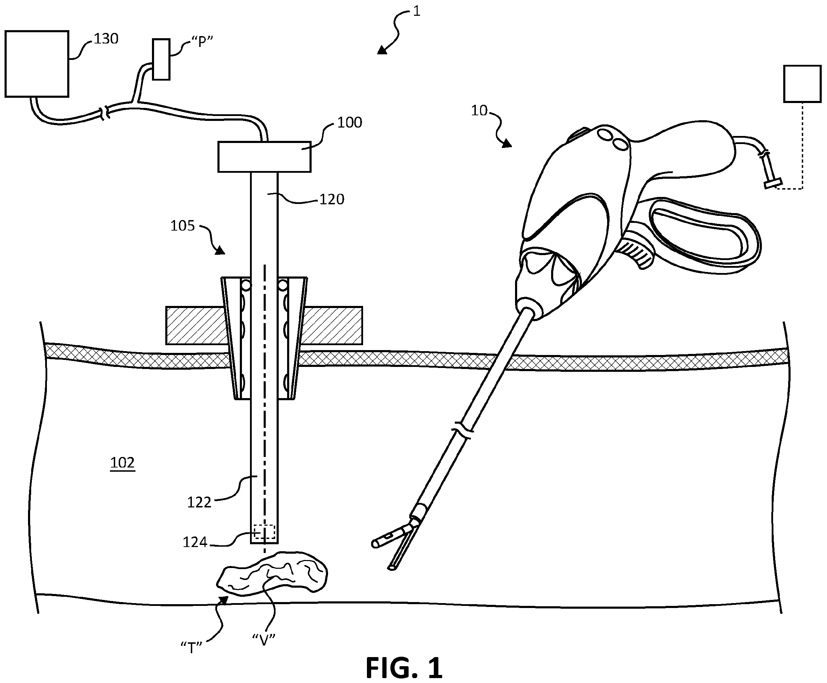

With reference to FIG. 1, the surgical imaging system 1 includes an endoscope 100 that may be introduced into a surgical site in a minimally-invasive manner utilizing a port assembly 105. The surgical imaging system 1 may be used during a surgical procedure involving the use of a surgical instrument, such as a bipolar forceps 10, for treating tissue in the surgical site. The port assembly 105 is adapted to allow access into a surgical site, such as, for example, a body cavity 102, e.g., to allow access of instruments therethrough, and may include sealing elements or mechanisms to seal the opening into the body cavity 102, e.g., to prevent the escape of insufflation gas. Although FIG. 1 depicts a bipolar forceps 10 for use in connection with endoscopic surgical procedures, the endoscope 100 and the port assembly 105, when operably coupled together, may be used with a variety of instruments depending on the type of surgery (e.g., surgical staplers, clip appliers, etc.).

For a detailed description of the construction and operation of an access port, reference may be made to U.S. Patent Application Publication No. 2016/0089181, filed on October 22, 2015, the entire contents of which are incorporated by reference herein.

The endoscope 100 generally includes an elongated shaft 120 having a distal end portion 122 and an imager 124, such as, for example, a camera or an imaging modality, configured to capture an image. The imager 124 may include a light for providing illumination and a camera chip. The imager 124 is disposed within the distal end portion 122 of the endoscope 100 and is operably coupled to a power source (not explicitly shown) via a transmission line coupled to the endoscope 100. In other embodiments, the imager 124 may be positioned at any suitable location of the endoscope 100.

The imager 124 may be any suitable imaging apparatus configured for still or moving imaging including, but not limited to, digital devices, such as charge-coupled device (CCD) camera, a complementary metal-oxide-semiconductor (CMOS) sensor, an active-pixel sensor (APS), and analog devices, such as a vidicon tube. In embodiments, the imager 124 may also include any suitable lens or optical apparatus (e.g., optical fiber) for transmitting light to a processor "P" of the surgical imaging system 1. The imager 124 may be in communication with a display device 130 for displaying the images captured thereby.

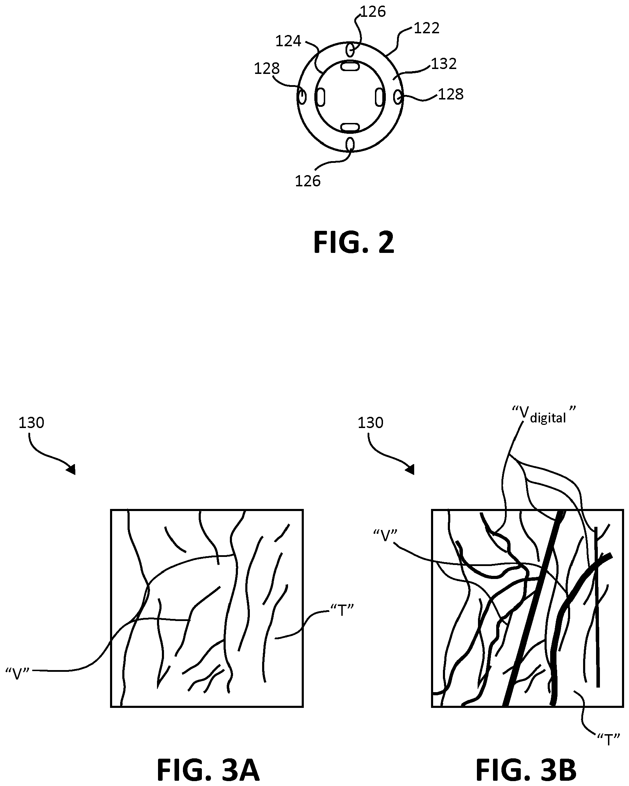

With reference to FIGS. 1 and 2, the endoscope 100 further includes one or more infrared transmitters 126, such as, for example, infrared light-emitting diodes ("IR-LEDs") or lasers for transmitting near-infrared light, and one or more infrared receivers 128 or sensors for receiving near-infrared light. The infrared receivers 128 may be an infrared sensitive optical sensor such as, for example, a charge coupled device ("CCD") sensor array, a complementary metal oxide semiconductor ("CMOS") sensor array, a phototransistor sensor array or the like. In embodiments, the infrared transmitters 126 and infrared receivers 128 may be designed as one sensor having both infrared transmission and reception capability.

The infrared transmitters and receivers 126, 128 are disposed in an annular array about the imager 124 and are attached to a distal-facing edge 132 of the distal end portion 122 of the endoscope 120. In embodiments, the infrared transmitters and receivers 126, 128 may be disposed at any suitable location of the endoscope 100. The infrared transmitters and receivers 126, 128 are in communication with the processor "P" of the surgical imaging system 1 for generating a digital image of vasculature targeted by the infrared transmitters 126, as will be described.

The processor "P" of the surgical imaging system 1 may be incorporated into the endoscope 100, the display 130, or be disposed at any other suitable location, such as, for example, a separate computer. The processor "P" is in communication with the infrared transmitters and receivers 126, 128. As such, the amount of infrared light transmitted to tissue by the infrared transmitters 126 and the amount of infrared light received by the infrared receivers 128 is known by the processor "P." The processor "P" is configured to use this data to generate a digital image of the vasculature targeted by the infrared transmitters 126.

The processor "P" may be operably connected to a memory, which may include transitory type memory (e.g., RAM) and/or non-transitory type memory (e.g., flash media, disk media, etc.). Those skilled in the art will appreciate that the processor "P" may be substituted by using any logic processor (e.g., control circuit) adapted to perform the calculations and/or set of instructions described herein including, but not limited to, field programmable gate arrays, digital signal processor, and combinations thereof. The processor "P" is capable of executing software instructions for processing the data received by the infrared receivers 126. The software instructions, which are executable by the processor "P", are stored in the memory.

The surgical imaging system 1 further includes the visual display 130 in communication with the imager 124 of the endoscope 100 and the processor "P." The display 130 may be a flat-panel display, such as, for example, an LCD, an LED screen, or the like. The display 130 is configured to display an image of tissue, including vasculature disposed underneath the tissue, captured by the imager 124 of the endoscope 100. The display 130 is further configured to display a digital image of the vasculature, generated by the processor "P" using the data received from the infrared receivers 126, over the actual image of the vasculature to give a clinician a clear view of where the vasculature is located relative to the outer surface of the tissue.

In operation, a minimally invasive surgical procedure may require knowledge of the location of vasculature "V" underneath tissue "T" either to avoid said vasculature or because said vasculature is the target of the operation. To locate and view the vasculature "V," the endoscope 100 is passed through the port assembly 105 to position the distal end portion 122 of the endoscope 100 adjacent the tissue "T." The imager 124 of the endoscope 100 captures an image (e.g., video or a still image) of the tissue "T" and displays the image of the tissue "T" on the display 130, as shown in FIG. 3A. Since most vasculature disposed underneath tissue is at least partially visible, the image of the tissue on the display 130 will, in most instances, also show the vasculature "V."

Concurrently with capturing the image of the tissue "T" with the imager 124, the infrared transmitters 126 of the endoscope 100 transmit infrared light toward the tissue "T." Due to the difference in infrared-absorption capability between tissue (e.g., muscle, skin, fat) and vasculature, most of the infrared light directed at the tissue "T" without vasculature "V" reflects back toward the endoscope 100, whereas most of the infrared light directed at the tissue "T" having the vasculature "V" disposed underneath gets absorbed by the vasculature "V." The infrared light that gets reflected by the tissue "T" is received by the infrared receivers 128, which transfer the data to the processor "P."

The processor "P," using the data received from the infrared receivers 126, locates/identifies the vasculature "V" and generates a digital image of the vasculature "V_{digital}." The processor "P" relays the digital image of the vasculature "V_{digital}" to the display 130, whereby the display 130 superimposes the digital image of the vasculature "V_{digital}" on the actual image of the vasculature "V" captured by the imager 124. The clinician, now with a better visualization of the vasculature "V," may more effectively navigate around the vasculature "V" or treat the vasculature "V" depending on the surgical procedure being performed.

For a more detailed description of vein identification and viewing technology, reference may be made to U.S. Patent Application Publication No. 2006/0020212, filed on July 26, 2004, and U.S. Patent No. 9,789,267, filed on May 21, 2015, the entire contents of each of which being incorporated by reference herein.

In embodiments, the digital image of the vasculature "V_{digital}" displayed on the display 130 may be a color different than the actual color of the vasculature "V." For example, the digital image of the vasculature "V_{digital}" may be displayed in yellow, green, blue, or any suitable color.

In embodiments, the surgical imaging system 1 may include a temperature sensor (not shown) for determining a temperature of the vasculature "V." The processor "P" or the display 130 may utilize the temperature of the vasculature "V" determined by the temperature sensor to adjust the color of the digital image of the vasculature "V_{digital}" based on the temperature of the vasculature "V." For example, if the temperature of the vasculature "V" is cooler than a known baseline temperature (e.g., 98.6°F) or range of temperatures (e.g. 95°F - 99°F), then the digital image of the vasculature "V_{digital}" may be displayed as blue, whereas if the temperature of the vasculature "V" is warmer than the known baseline temperature or range of temperatures, then the digital image of the vasculature "V_{digital}" may be displayed as yellow.

In embodiments, the components of the system 1 (e.g., the endoscope 100, the processor "P," and the display 130) may be incorporated into a portable, hand-held device.

It will be understood that various modifications may be made to the embodiments disclosed herein. Therefore, the above description should not be construed as limiting, but merely as exemplifications of various embodiments. Those skilled in the art will envision other modifications within the scope and spirit of the claims appended thereto.

The invention may be described by reference to the following numbered paragraphs:-
1. A surgical imaging system, comprising:
   an endoscope including:
      an elongated shaft having a distal end portion;
      an imager attached to the distal end portion and configured to capture an image of tissue;
      an infrared transmitter attached to the distal end portion of the shaft for transmitting infrared light to tissue; and
      an infrared receiver attached to the distal end portion of the shaft for receiving infrared light reflected by the tissue;
   a processor in communication with the infrared transmitter and the infrared receiver, the processor being configured to identify vasculature in the tissue and generate a digital image of the vasculature based on data received from the infrared receiver; and
   a display configured to display the image of the tissue captured by the imager and the digital image of the vasculature generated by the processor, wherein the digital image of the vasculature overlays the image of the tissue.
2. The surgical imaging system according to paragraph 1, wherein the image of the tissue captured by the imager includes an image of the vasculature, the display being configured to superimpose the digital image of the vasculature generated by the processor on the image of the vasculature captured by the imager.
3. The surgical imaging system according to paragraph 1, wherein the image of the vasculature is displayed in a color different than an actual color of the vasculature.
4. The surgical imaging system according to paragraph 3, wherein the color of the digital image of the vasculature changes based on a temperature of the vasculature.
5. A method of imaging vasculature during a surgical procedure, comprising:
   positioning a distal end portion of an endoscope adjacent tissue in a surgical site;
   capturing an image of the tissue with an imager attached to the distal end portion of the endoscope;
   transmitting infrared light to the tissue from an infrared transmitter attached to the distal end portion of the shaft; and
   receiving the infrared light that is reflected by the tissue into an infrared receiver that is attached to the distal end portion of the shaft;
   generating a digital image of vasculature in the tissue using data from the infrared receiver;
   displaying the image of the tissue captured by the imager on a display; and
   displaying the digital image of the vasculature on the display over the image of the tissue.
6. The method according to paragraph 5, further comprising identifying a location of the vasculature with a processor, wherein the processor generates the digital image of the vasculature using the data from the infrared receiver.
7. The method according to paragraph 5, wherein the image of the tissue captured by the imager includes an image of the vasculature, the display being configured to superimpose the digital image of the vasculature generated on the image of the vasculature captured by the imager.
8. The method according to paragraph 5, further comprising displaying the digital image of the vasculature on the display in a color different than an actual color of the vasculature.
9. The method according to paragraph 8, wherein the color of the digital image of the vasculature changes based on a temperature of the vasculature.

## Claims

1. A surgical imaging system, comprising:
an endoscope including:
an elongated shaft having a distal end portion;
an imager attached to the distal end portion and configured to capture an image of tissue;
an infrared transmitter attached to the distal end portion of the shaft for transmitting infrared light to tissue; and
an infrared receiver attached to the distal end portion of the shaft for receiving infrared light reflected by the tissue;
a processor in communication with the infrared transmitter and the infrared receiver, the processor being configured to identify vasculature in the tissue and generate a digital image of the vasculature based on data received from the infrared receiver; and
a display configured to display the image of the tissue captured by the imager and the digital image of the vasculature generated by the processor, wherein the digital image of the vasculature overlays the image of the tissue.

2. The surgical imaging system according to claim 1, wherein the image of the tissue captured by the imager includes an image of the vasculature, the display being configured to superimpose the digital image of the vasculature generated by the processor on the image of the vasculature captured by the imager.

3. The surgical imaging system according to claim 1 or claim 2, wherein the image of the vasculature is displayed in a color different than an actual color of the vasculature.

4. The surgical imaging system according to claim 3, wherein the color of the digital image of the vasculature changes based on a temperature of the vasculature.

5. A method of imaging vasculature during a surgical procedure, comprising:
positioning a distal end portion of an endoscope adjacent tissue in a surgical site;
capturing an image of the tissue with an imager attached to the distal end portion of the endoscope;
transmitting infrared light to the tissue from an infrared transmitter attached to the distal end portion of the shaft; and
receiving the infrared light that is reflected by the tissue into an infrared receiver that is attached to the distal end portion of the shaft;
generating a digital image of vasculature in the tissue using data from the infrared receiver;
displaying the image of the tissue captured by the imager on a display; and
displaying the digital image of the vasculature on the display over the image of the tissue.

6. The method according to claim 5, further comprising identifying a location of the vasculature with a processor, wherein the processor generates the digital image of the vasculature using the data from the infrared receiver.

7. The method according to claim 5 or claim 6, wherein the image of the tissue captured by the imager includes an image of the vasculature, the display being configured to superimpose the digital image of the vasculature generated on the image of the vasculature captured by the imager.

8. The method according to claim 5, further comprising displaying the digital image of the vasculature on the display in a color different than an actual color of the vasculature.

9. The method according to claim 8, wherein the color of the digital image of the vasculature changes based on a temperature of the vasculature.
